# EUROPEAN PATENT APPLICATION

(11) **EP 4 056 194 A1**
(43) Date of publication of application: **14.09.2022**
(21) Application number: 21209064.1
(22) Date of filing: 26.02.2016
(51) Int. Cl.: A61K 38/08, A61K 38/10, A61K 38/49, A61K 9/00, A61P 11/00

(54) **POLYPEPTIDE THERAPEUTICS AND USES THEREOF**

(30) Priority: 27.02.2015 US 201562126039 P
(62) Divisional of application: 16711063.4
(71) Applicant: Board of Regents, The University of Texas System, Austin, TX 78701 (US)
(72) Inventor: WILLIAMS, Robert, O., Austin, TX, 78746 (US); IDELL, Steven, Tyler, TX, 75703 (US); SHETTY, Sreerama, Tyler, TX, 75707 (US)
(74) Representative: Vossius & Partner Patentanwälte Rechtsanwälte mbB

(57) **Abstract**

Methods and composition for delivery of enzymes to a subject's airway. In some aspects, nebulized composition of enzymes, such as plasminogen activators are provided. In further aspects perfluorocarbon compositions comprising enzymes, such as plasminogen activators are provided. Compositions may, in some aspects, be used for the treatment of lung infections or acute lung injury, such as inhalational smoke induced acute lung injury (ISALI).

## Description

The present application claims the priority benefit of United States provisional application number 62/126,039, filed February 27, 2015, the entire contents of which are incorporated herein by reference.

### INCORPORATION OF SEQUENCE LISTING

The sequence listing that is contained in the file named "UTFBP1049WO_ST25.txt", which is 1 KB (as measured in Microsoft Windows^{®}) and was created on February 26, 2016, is filed herewith by electronic submission and is incorporated by reference herein.

### BACKGROUND OF THE INVENTION

### 1. Field of the Invention

The present invention relates generally to the field of molecular biology, drug delivery and medicine. More particularly, it concerns compositions and methods for the delivery of therapeutic polypeptide compositions to subjects, such as by delivery to the respiratory system.

### 2. Description of Related Art

During lung injury, p53 expression increases, inducing plasminogen activator inhibitor-1 (PAI-1) while inhibiting expression of urokinase-type plasminogen activator (uPA) and its receptor (uPAR), resulting in apoptosis of lung epithelial cells (LECs). The mechanism of injury involves cell surface signaling interactions between uPA, uPAR, caveolin-1 ("Cav-1") and β1-integrin (Shetty *et al.,* 2005). Compositions that modulate these interactions could be used in methods for inhibiting apoptosis of injured or damaged lung epithelial cells and for treating acute lung injury and consequent pulmonary fibrosis. Thus, there is a need for polypeptides that could be used to prevent or treat lung injury and, in particular, formulations and methods for therapeutic delivery of such polypeptides.

### SUMMARY OF THE INVENTION

In a first embodiment there is provided pharmaceutically acceptable formulation of a Cav-1 scaffolding domain polypeptide. For example, in some aspects, the Cav-1 scaffolding domain polypeptide is formulated for delivery to the respiratory system. Thus, in some aspects, there is provided a nebulized solution of a biologically active Cav-1 scaffolding domain polypeptide. In preferred aspects, the Cav-1 scaffolding domain polypeptide comprises the amino acid sequence of FTTFTVT ("CSP-7"; SEQ ID NO: 1), or a sequence having 1, 2 or 3 amino acid deletions, substitution or insertions relative to SEQ ID NO: 1. In still a further aspect, there is provided Cav-1 scaffolding domain polypeptide comprising the sequence of DGIWKASFTTFTVTKYWFYR (SEQ ID NO: 2), or a sequence having 1, 2, 3, 4, 5, 6, 7 or 8 amino acid deletions, substitution or insertions relative to SEQ ID NO: 2. In certain aspects, a Cav-1 scaffolding domain polypeptide of the embodiments is no more than 50, 45, 40, 35, 30, 25, 20, 15, 14, 13, 12, 11, 10, 9, 8 or 7 amino acids in length. In specific aspects, the Cav-1 scaffolding domain polypeptide consists of, or consists essentially of, the sequence of FTTFTVT (SEQ ID NO:1).

In a further embodiment there is provided pharmaceutically acceptable formulation polypeptide comprising the sequence of NYHYLESSMTALYTLGH (SEQ ID NO:3), or a sequence having 1, 2, 3, 4 or 5 amino acid deletions, substitution or insertions relative to SEQ ID NO: 3. In some aspects, the pharmaceutically acceptable formulation is formulated for delivery to the respiratory system. Thus, in some aspects, there is provided a nebulized solution of a biologically active polypeptide comprising the sequence of NYHYLESSMTALYTLGH (SEQ ID NO:3), or a sequence having 1, 2, 3, 4 or 5 amino acid deletions, substitution or insertions relative to SEQ ID NO: 3. In certain aspects, the polypeptide is no more than 50, 45, 40, 35, 30, 25, 20, 15, 14, 13, 12, 11, 10, 9, 8 or 7 amino acids in length.

Pharmaceutically acceptable formulations of the embodiments may include, without limitation, salts, buffers, preservatives, thickener, stabilizers and surfactants. In certain aspects, the formulations are aqueous formulations. In some cases, the formulations are lyophilized and may, in some cases, be solubilized in a solution prior to administration. In certain aspects, pharmaceutical formulation of the embodiments is essentially free of a cationic, anionic, zwitterionic or non-ionic surfactants surfactant. In preferred aspects, pharmaceutical formulations of the embodiments is filtered and/or sterilized. In specific aspects, a pharmaceutical formulation comprises a sterile saline or phosphate buffered saline (PBS) solution. In other aspects, the solution is essentially free of a pH buffering agent. In further aspects, a pharmaceutical formulation comprises a stabilizer. For example, the stabilizer can comprise, amino acids, such as glycine and lysine, carbohydrates or a lyoprotectant (e.g., dextrose, mannose, galactose, fructose, lactose, sucrose, maltose, sorbitol, or mannitol). In specific aspects, the pharmaceutical formulation consists essentially of a sterile saline solution and a biologically active polypeptide, such as Cav-1 scaffolding domain polypeptide. Further components for inclusion in pharmaceutical formulations of the embodiments are detailed herein below. In some aspects, a nebulized composition of the embodiments comprises a lyoprotectant (e.g., dextrose, mannose, galactose, fructose, lactose, sucrose, maltose, sorbitol, or mannitol) that is present in a ratio of 100:1 to 5:1; 100:1 to 10:1; 60:1 to 10:1; 50:1 to 20:1; or 40:1 to 20:1 weight/weight (w/w) relative to the Cav-1 scaffolding domain polypeptide. For example, the composition may comprise mannitol in a ratio of 40:1 to 20:1 w/w (e.g., about 30:1) relative to the Cav-1 scaffolding domain polypeptide.

Thus, in some, embodiments a nebulized composition is provided comprising a polypeptide of the embodiments. In some aspects, the nebulized solution is produced using a vibrating mesh nebulizer. The vibrating mesh nebulizer may be an active or a passive vibrating mesh nebulizer. In some aspects, the vibrating mesh nebulizer may be an Aeroneb^{®} Professional Nebulizer System or an EZ Breathe Atomizer. In further aspects, the nebulized solution is produced using a jet nebulizer or an ultrasonic nebulizer. In some aspects, a nebulized solution of the embodiments may have a median particle size of between about 2.5 µm and 100 µm, 2.5. µm and 50 µm, 2.5 µm and 20 µm, 2.5 µm and 8 µm, or 3.0 µm and 6 µm.

In some aspects, a pharmaceutical formulation of the embodiments, such a nebulized solution, further comprises a biologically active enzyme. The enzyme may be a plasminogen activator enzyme. The plasminogen activator enzyme is tissue plasminogen activator (tPA) or urokinase plasminogen activator (uPA) in some further aspects. The uPA may be a single chain uPA (scuPA).

In a further embodiment there is provided a composition for use in the treatment of treating or preventing a disease, injury or infection of the lungs (*e.g.,* a fibrotic condition of the lungs), said composition comprising a polypeptide of the embodiments in pharmaceutically acceptable carrier. In certain embodiment that composition is a nebulized composition, such a composition formulated for delivery to the airway.

In still a further embodiment there is provided a lyophilized composition comprising a biologically active caveolin-1 (Cav-1) scaffolding domain polypeptide and at least a first lyoprotectant agent, wherein the Cav-1 scaffolding domain polypeptide remains soluble when exposed to an aqueous solution. In some aspects, a lyophilized composition of the embodiments comprises a lyoprotectant (*e.g*., dextrose, mannose, galactose, fructose, lactose, sucrose, maltose, sorbitol, or mannitol) that is present in a ratio of 100:1 to 5:1; 100:1 to 10:1; 60:1 to 10:1; 50:1 to 20:1; or 40:1 to 20:1 weight/weight (w/w) relative to the Cav-1 scaffolding domain polypeptide. For example, the composition may comprise mannitol in a ratio of 40:1 to 20:1 w/w (e.g., about 30:1) relative to the Cav-1 scaffolding domain polypeptide.

In yet a further embodiment there is provided a method of treating or preventing a disease, injury or infection of the lungs (*e.g.,* a fibrotic condition of the lungs) in a subject comprising administering to the subject an effective amount polypeptide of the embodiments in a pharmaceutically acceptable carrier. For example, a method may comprise administering a biologically active Cav-1 scaffolding domain polypeptide, such as a polypeptide provided in a nebulized solution to the airway of the subject. In some aspects, the subject has an acute lung injury or infection or a chemical-induced lung injury. In specific aspects, the subject has plastic bronchitis, asthma, chronic obstructive airway/pulmonary (COPD), acute respiratory distress syndrome (ARDS) inhalational smoke induced acute lung injury (ISALI), bronchiectasis, inhalational toxin-induced airway disease (e.g., chlorine induced airways disease), exposure to mustard gas, exposure to particulate matter (e.g., silica dust), bronchiolitis obliterans, bronchiolitis obliterans organizing pneumonia, collagen vascular lung disease (e.g., from lupus, scleroderma or mixed connective tissue disease), interstitial lung disease (e.g., idiopathic pulmonary fibrosis or sarcoidosis), drug induced lung disease and accelerated pulmonary fibrosis (e.g., that occurs after acute lung injury including ARDS).

In some aspects, a method of administering a pharmaceutical formulation of the embodiments comprises nebulizing a solution comprising a polypeptide (such as a Cav-1 scaffolding domain polypeptide). The nebulizing may or may not comprise using an ultrasonic or jet nebulizer to nebulize the solution comprising the Cav-1 scaffolding domain polypeptide. In other aspects, the nebulizing comprises using a vibrating mesh nebulizer to nebulize the solution comprising the Cav-1 scaffolding domain polypeptide. The vibrating mesh nebulizer may be an active or a passive vibrating mesh nebulizer. In some aspects, the vibrating mesh nebulizer may be an Aeroneb^{®} Professional Nebulizer System or an EZ Breathe Atomizer.

In still further aspects, nebulizing a pharmaceutical formulation of the embodiments comprises obtaining a lyophilized polypeptide composition (*e.g*., comprising a Cav-1 scaffolding domain polypeptide), reconstituting the lyophilized enzyme composition in an aqueous solution to provide polypeptide solution, and nebulizing the polypeptide solution. In some aspects, a nebulized solution is administered according to the embodiments and aspects described above. In still further aspects, a biologically active plasminogen activator enzyme is additionally administered to the subject. The plasminogen activator enzyme may be administered in a nebulized solution to the airway of the subject. The plasminogen activator enzyme may be tPA or uPA. In specific aspects, the uPA may be a single chain uPA (scuPA).

In still a further embodiment there is provided a method of preparing a polypeptide, such as a biologically active Cav-1 scaffolding domain polypeptide, for administration to a subject's airway comprising nebulizing a solution comprising the polypeptide to provide a nebulized solution.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%. Most preferred is a composition in which no amount of the specified component can be detected with standard analytical methods.

The term "administering" refers to an administration that is oral, topical, intravenous, subcutaneous, transcutaneous, transdermal, intramuscular, intra-joint, parenteral, intra-arteriole, intradermal, intraventricular, intracranial, intraperitoneal, intralesional, intranasal, rectal, vaginal, by inhalation or via an implanted reservoir. The term "parenteral" includes subcutaneous, intravenous, intramuscular, intra-articular, intra-synovial, intrasternal, intrathecal, intrahepatic, intralesional, and intracranial injections or infusion techniques. In some embodiments, the administration is via inhalation of a nebulized composition.

The term "airway" refers herein to any portion of the respiratory tract including the upper respiratory tract, the respiratory airway, and the lungs. The upper respiratory tract includes the nose and nasal passages, mouth, and throat. The respiratory airway includes the larynx, trachea, bronchi and bronchioles. The lungs include the respiratory bronchioles, alveolar ducts, alveolar sacs and alveoli.

The terms "inhalational smoke induced acute lung injury" and "ISALI" are used interchangeably herein and refer to a form of acute lung injury (ALI) caused by smoke inhalation. ALI is also referred to as "mild ARDS." ALI can be defined by finding one or more of the following conditions in a subject: 1) bilateral pulmonary infiltrates on chest x-ray, 2) when measured by right heart catheterization as clinically indicated, pulmonary capillary wedge pressure < 18 mmHg (2.4 kPa), and 3) PaO₂/FiO₂ <300 mmHg (40 kPa). In some embodiments, treatment of ISALI includes treatment of one or more of the following conditions: reduced oxygenation, airway obstruction (including a severe airway obstruction), fibrinous airway casts or debris, and alveolar fibrin deposition.

The terms "nebulizing," "nebulized" and other grammatical variations, refer herein to the process of converting a liquid into small aerosol droplets. In some embodiments, the aerosol droplets have a median diameter of approximately 2-10 µm. In some embodiments, the aerosol droplets have a median diameter of approximately 2-4 µm.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1 -** A schematic illustration of CSP dispersion nebulization procedure.
**FIG. 2** - CSP-7 peptide content of compositions was measured following nebulization according to the varying preparations described in Example 1.
**FIG. 3** - Chemical assay data of CPS-7 mer after processing and nebulization given as % of peptide remaining.

### DESCRIPTION OF ILLUSTRATIVE EMBODIMENTS

### I. The Present Invention

Provided herein are pharmaceutical formulations of biologically active polypeptides, such as the Cav-1 scaffolding domain polypeptides. For example, in some aspects, the Cav-1 scaffolding domain polypeptide is formulated for delivery to the respiratory system. Polypeptides can be prepared for administration to a subject's airway by formulation in an aqueous solution and nebulizing the enzyme solution using a nebulizer, such as a vibrating mesh nebulizer. It is a surprising finding of the present studies detailed herein that nebulization of polypeptides, such Cav-1 scaffolding domain polypeptides, results in nebulized compositions that maintain a significant proportion of the polypeptide. Also provided herein is a method of treating lung injuries and diseases, by administering to the subject a therapeutically effective amount of a nebulized polypeptides, such as Cav-1 scaffolding domain polypeptides, *via* the airway.

### II. Biologically Active Polypeptides

Certain aspects of the embodiments concern biologically active polypeptides that can be used to treat and prevent infections, injuries or disease of the lungs. In particular, a polypeptide for use according to the embodiments can be comprise a sequence at least about 80%, 85%, 80%, 85%, 90%, 91%, 92%, 93%, 94%, 95%, 96%, 97%, 98% or 99% identical to SEQ ID NO: 1, SEQ ID NO: 2 or SEQ ID NO: 3. In certain aspects, a polypeptide the embodiments may comprise 1, 2, 3 or more amino acid substitutions, deletions or insertions relative to the sequences of SEQ ID NO: 1; SEQ ID NO: 2 or SEQ ID NO: 3. Moreover, in some aspects, the polypeptide is no more than 50, 45, 40, 35, 30, 25, 20, 15, 14, 13, 12, 11, 10, 9, 8 or 7 amino acids in length. In still further aspects, a polypeptide of the embodiments comprises 1, 2, 3 or more repeats of sequence of SEQ ID NO: 1, SEQ ID NO: 2 or SEQ ID NO: 3.

The term "identity" or "homology" shall be construed to mean the percentage of amino acid residues in the candidate sequence that are identical with the residue of a corresponding sequence to which it is compared, after aligning the sequences and introducing gaps, if necessary to achieve the maximum percent identity for the entire sequence, and not considering any conservative substitutions as part of the sequence identity. Neither N- or C-terminal extensions nor insertions shall be construed as reducing identity or homology. Methods and computer programs for the alignment are well known in the art. Sequence identity may be measured using sequence analysis software.

The term "polypeptide" is used in its broadest sense to refer to a compound of two or more subunit amino acids, amino acid analogs, or peptidomimetics. The subunits may be linked by peptide bonds. In another embodiment, the subunit may be linked by other bonds, e.g. ester, ether, etc. As used herein the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is commonly called a polypeptide or a protein.

A polynucleotide or polynucleotide region (or a polypeptide or polypeptide region) has a certain percentage (for example, 80%, 85%, 90%, or 95%) of "sequence identity" or "homology" to another sequence means that, when aligned, that percentage of bases (or amino acids) are the same in comparing the two sequences. This alignment and the percent homology or sequence identity can be determined using software programs known in the art, for example those described in CURRENT PROTOCOLS IN MOLECULAR BIOLOGY (F. M. Ausubel et al., eds., 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+SwissProtein+SPupdate+PIR.

In some aspects, amino acid substitutions can be made in a polypeptide at one or more positions wherein the substitution is for an amino acid having a similar hydrophilicity. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Thus such conservative substitution can be made in a polypeptide and will likely only have minor effects on their activity. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (0.5); histidine - 0.5); cysteine (-1.0); methionine (-1.3); valine (-1.5); leucine (-1.8); isoleucine (-1.8); tyrosine (-2.3); phenylalanine (-2.5); tryptophan (-3.4). These values can be used as a guide and thus substitution of amino acids whose hydrophilicity values are within ±2 are preferred, those that are within ±1 are particularly preferred, and those within ±0.5 are even more particularly preferred. Thus, any of the polypeptides described herein may be modified by the substitution of an amino acid, for different, but homologous amino acid with a similar hydrophilicity value. Amino acids with hydrophilicities within +/- 1.0, or +/- 0.5 points are considered homologous.

### Conjugates

Compositions and methods of the present invention involve biologically active polypeptides, such Cav-1 scaffolding domain polypeptides, which maybe conjugated with heterologous peptide segments or polymers, such as polyethylene glycol. In further aspects, the engineered polypeptides may be linked to PEG to increase the hydrodynamic radius of the enzyme and hence increase the serum persistence. In certain aspects, the disclosed polypeptide may be conjugated to any targeting agent, such as a ligand having the ability to specifically and stably bind to an external receptor or binding site on a tumor cell (U.S. Patent Publ. 2009/0304666).

### A. Fusion Proteins

Certain embodiments of the present invention concern fusion proteins. These molecules may have the polypeptide of the embodiments linked at the N- or C-terminus to a heterologous domain. For example, fusions may also employ leader sequences from other species to permit the recombinant expression of a protein in a heterologous host. Another useful fusion includes the addition of a protein affinity tag, such as a serum albumin affinity tag or six histidine residues, or an immunologically active domain, such as an antibody epitope, preferably cleavable, to facilitate purification of the fusion protein. Non-limiting affinity tags include polyhistidine, chitin binding protein (CBP), maltose binding protein (MBP), and glutathione-S-transferase (GST).

In a particular embodiment, the polypeptide of the embodiments may be linked to a peptide that increases the *in vivo* half-life, such as an XTEN polypeptide (Schellenberger *et al.,* 2009), IgG Fc domain, albumin, or albumin binding peptide.

Methods of generating fusion proteins are well known to those of skill in the art. Such proteins can be produced, for example, by *de novo* synthesis of the complete fusion protein, or by attachment of the DNA sequence encoding the heterologous domain, followed by expression of the intact fusion protein.

Production of fusion proteins that recover the functional activities of the parent proteins may be facilitated by connecting genes with a bridging DNA segment encoding a peptide linker that is spliced between the polypeptides connected in tandem. The linker would be of sufficient length to allow proper folding of the resulting fusion protein.

### B. Linkers

In certain embodiments, the polypeptide of the embodiments may be chemically conjugated using bifunctional cross-linking reagents or fused at the protein level with peptide linkers.

Bifunctional cross-linking reagents have been extensively used for a variety of purposes, including preparation of affinity matrices, modification and stabilization of diverse structures, identification of ligand and receptor binding sites, and structural studies. Suitable peptide linkers may also be used to link the polypeptide of the embodiments, such as Gly-Ser linkers.

Homobifunctional reagents that carry two identical functional groups proved to be highly efficient in inducing cross-linking between identical and different macromolecules or subunits of a macromolecule, and linking of polypeptide ligands to their specific binding sites. Heterobifunctional reagents contain two different functional groups. By taking advantage of the differential reactivities of the two different functional groups, cross-linking can be controlled both selectively and sequentially. The bifunctional cross-linking reagents can be divided according to the specificity of their functional groups, *e.g*., amino-, sulfhydryl-, guanidine-, indole-, carboxyl-specific groups. Of these, reagents directed to free amino groups have become especially popular because of their commercial availability, ease of synthesis, and the mild reaction conditions under which they can be applied.

A majority of heterobifunctional cross-linking reagents contain a primary amine-reactive group and a thiol-reactive group. In another example, heterobifunctional cross-linking reagents and methods of using the cross-linking reagents are described (U.S. Pat. No. 5,889,155, specifically incorporated herein by reference in its entirety). The cross-linking reagents combine a nucleophilic hydrazide residue with an electrophilic maleimide residue, allowing coupling, in one example, of aldehydes to free thiols. The cross-linking reagent can be modified to cross-link various functional groups.

Additionally, any other linking/coupling agents and/or mechanisms known to those of skill in the art may be used to combine polypeptides of the embodiments, such as, for example, antibody-antigen interaction, avidin biotin linkages, amide linkages, ester linkages, thioester linkages, ether linkages, thioether linkages, phosphoester linkages, phosphoramide linkages, anhydride linkages, disulfide linkages, ionic and hydrophobic interactions, bispecific antibodies and antibody fragments, or combinations thereof.

It is preferred that a cross-linker having reasonable stability in blood will be employed. Numerous types of disulfide-bond containing linkers are known that can be successfully employed to conjugate targeting and therapeutic/preventative agents. Linkers that contain a disulfide bond that is sterically hindered may prove to give greater stability *in vivo.* These linkers are thus one group of linking agents.

In addition to hindered cross-linkers, non-hindered linkers also can be employed in accordance herewith. Other useful cross-linkers, not considered to contain or generate a protected disulfide, include SATA, SPDP, and 2-iminothiolane (Wawrzynczak and Thorpe, 1987). The use of such cross-linkers is well understood in the art. Another embodiment involves the use of flexible linkers.

Once chemically conjugated, the peptide generally will be purified to separate the conjugate from unconjugated agents and from other contaminants. A large number of purification techniques are available for use in providing conjugates of a sufficient degree of purity to render them clinically useful.

Purification methods based upon size separation, such as gel filtration, gel permeation, or high performance liquid chromatography, will generally be of most use. Other chromatographic techniques, such as Blue-Sepharose separation, may also be used. Conventional methods to purify the fusion proteins from inclusion bodies may be useful, such as using weak detergents, such as sodium N-lauroyl-sarcosine (SLS).

### C. PEGylation

In certain aspects, methods and compositions of the embodiments related to PEGylation of disclosed polypeptides. PEGylation is the process of covalent attachment of poly(ethylene glycol) polymer chains to another molecule, normally a drug or therapeutic protein. PEGylation is routinely achieved by incubation of a reactive derivative of PEG with the target macromolecule. The covalent attachment of PEG to a drug or therapeutic protein can "mask" the agent from the host's immune system (reduced immunogenicity and antigenicity) or increase the hydrodynamic size (size in solution) of the agent, which prolongs its circulatory time by reducing renal clearance. PEGylation can also provide water solubility to hydrophobic drugs and proteins.

The first step of the PEGylation is the suitable functionalization of the PEG polymer at one or both terminals. PEGs that are activated at each terminus with the same reactive moiety are known as "homobifunctional," whereas if the functional groups present are different, then the PEG derivative is referred as "heterobifunctional" or "heterofunctional." The chemically active or activated derivatives of the PEG polymer are prepared to attach the PEG to the desired molecule.

The choice of the suitable functional group for the PEG derivative is based on the type of available reactive group on the molecule that will be coupled to the PEG. For proteins, typical reactive amino acids include lysine, cysteine, histidine, arginine, aspartic acid, glutamic acid, serine, threonine, and tyrosine. The N-terminal amino group and the C-terminal carboxylic acid can also be used.

The techniques used to form first generation PEG derivatives are generally reacting the PEG polymer with a group that is reactive with hydroxyl groups, typically anhydrides, acid chlorides, chloroformates, and carbonates. In the second generation PEGylation chemistry more efficient functional groups, such as aldehyde, esters, amides, *etc.,* are made available for conjugation.

As applications of PEGylation have become more and more advanced and sophisticated, there has been an increase in need for heterobifunctional PEGs for conjugation. These heterobifunctional PEGs are very useful in linking two entities, where a hydrophilic, flexible, and biocompatible spacer is needed. Preferred end groups for heterobifunctional PEGs are maleimide, vinyl sulfones, pyridyl disulfide, amine, carboxylic acids, and NHS esters.

The most common modification agents, or linkers, are based on methoxy PEG (mPEG) molecules. Their activity depends on adding a protein-modifying group to the alcohol end. In some instances polyethylene glycol (PEG diol) is used as the precursor molecule. The diol is subsequently modified at both ends in order to make a hetero- or homo-dimeric PEG-linked molecule.

Proteins are generally PEGylated at nucleophilic sites, such as unprotonated thiols (cysteinyl residues) or amino groups. Examples of cysteinyl-specific modification reagents include PEG maleimide, PEG iodoacetate, PEG thiols, and PEG vinylsulfone. All four are strongly cysteinyl-specific under mild conditions and neutral to slightly alkaline pH but each has some drawbacks. The thioether formed with the maleimides can be somewhat unstable under alkaline conditions so there may be some limitation to formulation options with this linker. The carbamothioate linkage formed with iodo PEGs is more stable, but free iodine can modify tyrosine residues under some conditions. PEG thiols form disulfide bonds with protein thiols, but this linkage can also be unstable under alkaline conditions. PEG-vinylsulfone reactivity is relatively slow compared to maleimide and iodo PEG; however, the thioether linkage formed is quite stable. Its slower reaction rate also can make the PEG-vinylsulfone reaction easier to control.

Site-specific PEGylation at native cysteinyl residues is seldom carried out, since these residues are usually in the form of disulfide bonds or are required for biological activity. On the other hand, site-directed mutagenesis can be used to incorporate cysteinyl PEGylation sites for thiol-specific linkers. The cysteine mutation must be designed such that it is accessible to the PEGylation reagent and is still biologically active after PEGylation.

Amine-specific modification agents include PEG NHS ester, PEG tresylate, PEG aldehyde, PEG isothiocyanate, and several others. All react under mild conditions and are very specific for amino groups. The PEG NHS ester is probably one of the more reactive agents; however, its high reactivity can make the PEGylation reaction difficult to control on a large scale. PEG aldehyde forms an imine with the amino group, which is then reduced to a secondary amine with sodium cyanoborohydride. Unlike sodium borohydride, sodium cyanoborohydride will not reduce disulfide bonds. However, this chemical is highly toxic and must be handled cautiously, particularly at lower pH where it becomes volatile.

Due to the multiple lysine residues on most proteins, site-specific PEGylation can be a challenge. Fortunately, because these reagents react with unprotonated amino groups, it is possible to direct the PEGylation to lower-pK amino groups by performing the reaction at a lower pH. Generally the pK of the alpha-amino group is 1-2 pH units lower than the epsilon-amino group of lysine residues. By PEGylating the molecule at pH 7 or below, high selectivity for the N-terminus frequently can be attained. However, this is only feasible if the N-terminal portion of the protein is not required for biological activity. Still, the pharmacokinetic benefits from PEGylation frequently outweigh a significant loss of *in vitro* bioactivity, resulting in a product with much greater *in vivo* bioactivity regardless of PEGylation chemistry.

There are several parameters to consider when developing a PEGylation procedure. Fortunately, there are usually no more than four or five key parameters. The "design of experiments" approach to optimization of PEGylation conditions can be very useful. For thiol-specific PEGylation reactions, parameters to consider include: protein concentration, PEG-to-protein ratio (on a molar basis), temperature, pH, reaction time, and in some instances, the exclusion of oxygen. (Oxygen can contribute to intermolecular disulfide formation by the protein, which will reduce the yield of the PEGylated product.) The same factors should be considered (with the exception of oxygen) for amine-specific modification except that pH may be even more critical, particularly when targeting the N-terminal amino group.

For both amine- and thiol-specific modifications, the reaction conditions may affect the stability of the protein. This may limit the temperature, protein concentration, and pH. In addition, the reactivity of the PEG linker should be known before starting the PEGylation reaction. For example, if the PEGylation agent is only 70 percent active, the amount of PEG used should ensure that only active PEG molecules are counted in the protein-to-PEG reaction stoichiometry.

### III. Pharmaceutical Compositions

It is contemplated that polypeptides of the embodiments, such as Cav-1 scaffolding domain polypeptides, can be administered systemically or locally to inhibit cell apoptosis and for the treatment and prevention damage to lung tissues. They can be administered intravenously, intrathecally, and/or intraperitoneally. In preferred aspects, the polypeptides are delivered locally to the airway, such as administration of a nebulized formulation. They can be administered alone or in combination with anti-fibrotic compounds.

It is not intended that the present invention be limited by the particular nature of the therapeutic preparation. For example, such compositions can be provided in formulations together with physiologically tolerable liquid, gel, or solid carriers, diluents, and excipients. These therapeutic preparations can be administered to mammals for veterinary use, such as with domestic animals, and clinical use in humans in a manner similar to other therapeutic agents. In general, the dosage required for therapeutic efficacy will vary according to the type of use and mode of administration, as well as the particularized requirements of individual subjects.

Such compositions are typically prepared as liquid solutions or suspensions, as injectables. Suitable diluents and excipients are, for example, water, saline, dextrose, glycerol, or the like, and combinations thereof. In addition, if desired, the compositions may contain minor amounts of auxiliary substances, such as wetting or emulsifying agents, stabilizing agents, or pH buffering agents.

Where clinical applications are contemplated, it may be necessary to prepare pharmaceutical compositions comprising proteins, antibodies, and drugs in a form appropriate for the intended application. Generally, pharmaceutical compositions may comprise an effective amount of one or more of the polypeptides of the embodiments or additional agents dissolved or dispersed in a pharmaceutically acceptable carrier. The phrases "pharmaceutical or pharmacologically acceptable" refers to molecular entities and compositions that do not produce an adverse, allergic, or other untoward reaction when administered to an animal, such as, for example, a human, as appropriate. The preparation of a pharmaceutical composition that contains at least one polypeptide of the embodiments isolated by the method disclosed herein, or additional active ingredient will be known to those of skill in the art in light of the present disclosure, as exemplified by Remington's Pharmaceutical Sciences, 18th Ed., 1990, incorporated herein by reference. Moreover, for animal (e.g., human) administration, it will be understood that preparations should meet sterility, pyrogenicity, general safety, and purity standards as required by the FDA Office of Biological Standards.

As used herein, "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed., 1990, incorporated herein by reference). Except insofar as any conventional carrier is incompatible with the active ingredient, its use in the pharmaceutical compositions is contemplated.

Certain embodiments of the present invention may comprise different types of carriers depending on whether it is to be administered in solid, liquid, or aerosol form, and whether it needs to be sterile for the route of administration, such as injection. The compositions can be administered intravenously, intradermally, transdermally, intrathecally, intraarterially, intraperitoneally, intranasally, intravaginally, intrarectally, intramuscularly, subcutaneously, mucosally, orally, topically, locally, by inhalation (*e.g*., inhalation of a nebulized formulation), by injection, by infusion, by continuous infusion, by localized perfusion bathing target cells directly, *via* a catheter, *via* a lavage, in lipid compositions (*e.g.,* liposomes), or by other methods or any combination of the forgoing as would be known to one of ordinary skill in the art (see, for example, Remington's Pharmaceutical Sciences, 18th Ed., 1990, incorporated herein by reference).

The modified polypeptides may be formulated into a composition in a free base, neutral, or salt form. Pharmaceutically acceptable salts include the acid addition salts, *e.g*., those formed with the free amino groups of a proteinaceous composition, or which are formed with inorganic acids, such as, for example, hydrochloric or phosphoric acids, or such organic acids as acetic, oxalic, tartaric, or mandelic acid. Salts formed with the free carboxyl groups can also be derived from inorganic bases, such as, for example, sodium, potassium, ammonium, calcium, or ferric hydroxides; or such organic bases as isopropylamine, trimethylamine, histidine, or procaine. Upon formulation, solutions will be administered in a manner compatible with the dosage formulation and in such amount as is therapeutically effective. The formulations are easily administered in a variety of dosage forms, such as formulated for parenteral administrations, such as injectable solutions, or aerosols for delivery to the lungs, or formulated for alimentary administrations, such as drug release capsules and the like.

Further in accordance with certain aspects of the present invention, the composition suitable for administration may be provided in a pharmaceutically acceptable carrier with or without an inert diluent. The carrier should be assimilable and includes liquid, semi-solid, *i.e.,* pastes, or solid carriers. Except insofar as any conventional media, agent, diluent, or carrier is detrimental to the recipient or to the therapeutic effectiveness of a composition contained therein, its use in administrable composition for use in practicing the methods is appropriate. Examples of carriers or diluents include fats, oils, water, saline solutions, lipids, liposomes, resins, binders, fillers, and the like, or combinations thereof. The composition may also comprise various antioxidants to retard oxidation of one or more component. Additionally, the prevention of the action of microorganisms can be brought about by preservatives, such as various antibacterial and antifungal agents, including but not limited to parabens (*e.g*., methylparabens, propylparabens), chlorobutanol, phenol, sorbic acid, thimerosal or combinations thereof.

In accordance with certain aspects of the present invention, the composition is combined with the carrier in any convenient and practical manner, *i.e.,* by solution, suspension, emulsification, admixture, encapsulation, absorption, and the like. Such procedures are routine for those skilled in the art.

In a specific embodiment of the present invention, the composition is combined or mixed thoroughly with a semi-solid or solid carrier. The mixing can be carried out in any convenient manner, such as grinding. Stabilizing agents can be also added in the mixing process in order to protect the composition from loss of therapeutic activity, *i.e.,* denaturation in the stomach. Examples of stabilizers for use in a composition include buffers, amino acids, such as glycine and lysine, carbohydrates or lyoprotectants, such as dextrose, mannose, galactose, fructose, lactose, sucrose, maltose, sorbitol, mannitol, *etc.*

In further embodiments, the present invention may concern the use of a pharmaceutical lipid vehicle composition that includes polypeptides of the embodiments, one or more lipids, and an aqueous solvent. As used herein, the term "lipid" will be defined to include any of a broad range of substances that is characteristically insoluble in water and extractable with an organic solvent. This broad class of compounds is well known to those of skill in the art, and as the term "lipid" is used herein, it is not limited to any particular structure. Examples include compounds that contain long-chain aliphatic hydrocarbons and their derivatives. A lipid may be naturally occurring or synthetic (*i.e.,* designed or produced by man). However, a lipid is usually a biological substance. Biological lipids are well known in the art, and include for example, neutral fats, phospholipids, phosphoglycerides, steroids, terpenes, lysolipids, glycosphingolipids, glycolipids, sulphatides, lipids with ether- and ester-linked fatty acids, polymerizable lipids, and combinations thereof. Of course, compounds other than those specifically described herein that are understood by one of skill in the art as lipids are also encompassed by the compositions and methods.

One of ordinary skill in the art would be familiar with the range of techniques that can be employed for dispersing a composition in a lipid vehicle. For example, the polypeptides of the embodiments may be dispersed in a solution containing a lipid, dissolved with a lipid, emulsified with a lipid, mixed with a lipid, combined with a lipid, covalently bonded to a lipid, contained as a suspension in a lipid, contained or complexed with a micelle or liposome, or otherwise associated with a lipid or lipid structure by any means known to those of ordinary skill in the art. The dispersion may or may not result in the formation of liposomes.

The actual dosage amount of a composition administered to an animal patient can be determined by physical and physiological factors, such as body weight, severity of condition, the type of disease being treated, previous or concurrent therapeutic interventions, idiopathy of the patient, and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, an active compound may comprise between about 2% to about 75% of the weight of the unit, or between about 25% to about 60%, for example, and any range derivable therein. Naturally, the amount of active compound(s) in each therapeutically useful composition may be prepared in such a way that a suitable dosage will be obtained in any given unit dose of the compound. Factors, such as solubility, bioavailability, biological half-life, route of administration, product shelf life, as well as other pharmacological considerations, will be contemplated by one skilled in the art of preparing such pharmaceutical formulations, and as such, a variety of dosages and treatment regimens may be desirable.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 milligram/kg/body weight or more per administration, and any range derivable therein. In non-limiting examples of a derivable range from the numbers listed herein, a range of about 5 milligram/kg/body weight to about 100 milligram/kg/body weight, about 5 microgram/kg/body weight to about 500 milligram/kg/body weight, *etc.,* can be administered, based on the numbers described above.

In some aspects, a pharmaceutical formulation comprises one or more surfactant. Surfactants used in accordance with the disclosed methods include ionic and non-ionic surfactants. Representative non-ionic surfactants include polysorbates such as TWEEN^{®}-20 and TWEEN-80^{®} surfactants (ICI Americas Inc. of Bridgewater, N.J.); poloxamers (e.g., poloxamer 188); TRITON^{®} surfactants (Sigma of St. Louis, Mo.); sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palnidopropyl-, or(e.g., lauroamidopropyl); myristamidopropyl-, palmidopropyl-, or isostearamidopropyl-dimethylamine; sodium methyl cocoyl-, or disodium methyl oleyl-taurate; MONAQUAT^{™} surfactants (Mona Industries Inc. of Paterson, N.J.); polyethyl glycol; polypropyl glycol; block copolymers of ethylene and propylene glycol such as PLURONIC^{®} surfactants (BASF of Mt. Olive, N.J.); oligo (ethylene oxide) alkyl ethers; alkyl (thio) glucosides, alkyl maltosides; and phospholipids. For example, the surfactant can be present in a formulation in an amount from about 0.01% to about 0.5% (weight of surfactant relative to total weight of other solid components of the formulation; "w/w"), from about 0.03% to about 0.5% (w/w), from about 0.05% to about 0.5% (w/w), or from about 0.1% to about 0.5% (w/w). However, in further aspects, a pharmaceutical formulation of the embodiments is essentially free of non-ionic surfactants or essentially free of all surfactants.

Further provided herein is a composition comprising a polypeptide of the embodiments and a perfluorocarbon (PFC). In some embodiments, the polypeptide in the composition is selected from one of the polypeptides of SEQ ID NO: 1-3. In further embodiments, the PFC in the composition is selected from perfluorodecalin, perfluoro-1,3-dimethylcyclohexane, FC-75, perfluorooctane and perfluoro-octylbromide. In some aspects, PFC is or comprises a PFC having a cycloalkyl group, such as perfluorodecalin, perfluoro-1,3-dimethylcyclohexane or FC-75. It should be understood that the plasminogen activator and PFC can be in any ratio or concentration. In some embodiments, the composition comprises a plasminogen activator at a concentration of approximately 0.005-0.040 mg/mL of PFC.

Still further provided is a method of treating lung injury or disease (*e.g*., inhalational smoke induced acute lung injury (ISALI)) in a subject comprising administering to the subject a therapeutically effective amount of a composition comprising a polypeptide of the embodiments and a PFC. Accordingly, in some embodiments a method of administering a polypeptide and PFC composition is provided, wherein the PFC in the composition is selected from perfluorodecalin and perfluoro-octylbromide.

The terms "perfluorocarbon" and "PFC" are used interchangeably and refer herein to an organofluorine compound that contains predominantly carbon and fluorine. It should be understood that the term "perfluorocarbon" is meant to include highly fluorinated molecules that contain molecules in addition to carbon and fluorine, and are commonly referred to as fluorocarbons. Examples of perfluorocarbons include, but are not limited to, perfluorodecalin, perfluoro-octylbromide, FC 77, PF 5060 and Rimar 101. PFCs used according to the present invention share similar physicochemical properties with respect to gas solubility, density and surface tension but may differ with respect to radio-opacity and kinematic viscosity which could have an impact on visualization and mobility of airway casts during debridement. Each listed perfluorocarbon includes all relevant isomers such as stereoisomers, enantiomers, and diastereomers.

### IV. Aerosol Dispersion and Nebulizing Devices

The formulations can be aerosolized using any suitable device, including but not limited to a jet nebulizer, an ultrasonic nebulizer, a metered dose inhaler (MDI), and a device for aerosolization of liquids by forced passage through a jet or nozzle (*e.g*., AERX^{®} drug delivery devices by Aradigm of Hayward, Calif.). For delivery of a formulation to a subject, as described further herein below, an pulmonary delivery device can also include a ventilator, optionally in combination with a mask, mouthpiece, mist inhalation apparatus, and/or a platform that guides users to inhale correctly and automatically deliver the drug at the right time in the breath. Representative aerosolization devices that can be used in accordance with the methods of the present invention include but are not limited to those described in U.S. Pat. Nos. 6,357,671; 6,354,516; 6,241,159; 6,044,841; 6,041,776; 6,016,974; 5,823,179; 5,797,389; 5,660,166; 5,355,872; 5,284,133; and 5,277,175 and U.S. Published Patent Application Nos. 20020020412 and 20020020409.

Using a jet nebulizer, compressed gas from a compressor or hospital air line is passed through a narrow constriction known as a jet. This creates an area of low pressure, and liquid medication from a reservoir is drawn up through a feed tube and fragmented into droplets by the air stream. Only the smallest drops leave the nebulizer directly, while the majority impact on baffles and walls and are returned to the reservoir. Consequently, the time required to perform jet nebulization varies according to the volume of the composition to be nebulized, among other factors, and such time can readily be adjusted by one of skill in the art.

A metered dose inhalator (MDI) can be used to deliver a composition of the invention in a more concentrated form than typically delivered using a nebulizer. For optimal effect, MDI delivery systems require proper administration technique, which includes coordinated actuation of aerosol delivery with inhalation, a slow inhalation of about 0.5-0.75 liters per second, a deep breath approaching inspiratory capacity inhalation, and at least 4 seconds of breath holding. Pulmonary delivery using a MDI is convenient and suitable when the treatment benefits from a relatively short treatment time and low cost. Optionally, a formulation can be heated to about 25° C. to about 90° C. during nebulization to promote effective droplet formation and subsequent delivery. See e.g., U.S. Pat. No. 5,299,566.

Aerosol compositions of the embodiments comprise droplets of the composition that are a suitable size for efficient delivery within the lung. In some cases, a surfactant formulation is delivered to lung bronchi, more preferably to bronchioles, still more preferably to alveolar ducts, and still more preferably to alveoli. Aerosol droplets are typically less than about 15 µm in diameter, less than about 10 µm in diameter, less than about 5 µm in diameter, or less than about 2 µm in diameter. For efficient delivery to alveolar bronchi of a human subject, an aerosol composition may preferably comprises droplets having a diameter of about 1 µm to about 5 µm.

Droplet size can be assessed using techniques known in the art, for example cascade, impaction, laser diffraction, and optical patternation. See McLean et al. (2000) Anal Chem 72:4796-804, Fults et al. (1991) J Pharm Pharmacol 43:726-8, and Vecellio None et al. (2001) J Aerosol Med 14:107-14.

Protein stability following aerosolization can be assessed using known techniques in the art, including size exclusion chromatography; electrophoretic techniques; spectroscopic techniques such as UV spectroscopy and circular dichroism spectroscopy, and protein activity (measured in vitro or in vivo). To perform in vitro assays of protein stability, an aerosol composition can be collected and then distilled or absorbed onto a filter. To perform in vivo assays, or for pulmonary administration of a composition to a subject, a device for aerosolization is adapted for inhalation by the subject. For example, protein stability can be assessed by determining the level of protein aggregation. Preferably, an aerosol composition of the invention is substantially free of protein aggregates. The presence of soluble aggregates can be determined qualitatively using DLS (DynaPro-801TC, ProteinSolutions Inc. of Charlottesville, Va.) and/or by UV spectrophotometry.

The term "vibrating mesh nebulizer" refers herein to any nebulizer that operates on the general principle of using a vibrating mesh or plate with multiple aperatures (an aperture plate) to generate a fine-particle, low-velocity aerosol. Some nebulizers may contain a mesh/membrane with between 1000 and 7000 holes, which mesh/membrane vibrates at the top of a liquid reservoir (see, *e.g.,* U.S. Patent Publn. 20090134235 and Waldrep and Dhand 2008, each incorporated herein by reference). In some embodiments, the vibrating mesh nebulizer is an AERONEB^{®} Professional Nebulizer, Omron MICROAIR^{®}, Pari EFLOW^{®} or an EZ Breathe Atomizer. In some aspects, a vibrating mesh nebulizer has a vibrating frequency of between about 50-250 kHz, 75-200 kHz 100-150 kHz or about 120 kHz. These devices have a high efficiency of delivering aerosol to the lung and the volume of liquid remaining in these devices is minimal, which is an advantage for expensive and potent compounds like plasminogen activators.

In certain aspects, a nebulized composition of the embodiments is produced using a vibrating mesh nebulizer. For example, the composition can be produced with an active vibrating mesh nebulizer (*e.g.,* an Aeroneb^{®} Professional Nebulizer System). Descriptions of such system and there operation can be found, for instance, in U.S. Patents Nos. 6,921,020; 6,926,208; 6,968,840; 6,978,941; 7,040,549; 7,083,112; 7,104,463; and 7,360,536, each of which is incorporated herein by reference in its entirety. In yet further aspects, a composition of the embodiments can be produced with a passive vibrating mesh nebulizer, such as the Omron MicroAir^{®} or the EZ Breathe Atomizer.

### V. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Peptide Nebulization

*Materials* - Peptides were obtained from The University of Texas Health Science Center at Tyler and stored at -80°C. The peptides were either CSP, CSP-7, or scrambled. Two types of liquid media were used, both stored at room temperature. Dulbecco's Phosphate Buffer Solution (DPBS), manufactured by Gibco, Lot 14190-144, was purchased from Biostore at UT-Austin. The components of DPBS are shown in Table 1 below. Sterile Normal Saline (NS) (Packaged in 10mL vials and containing 0.9% w/v of sodium chloride), manufactured by APP Pharmaceuticals, LLC., NDC 63323-186-10, Lot 6008039, was purchased from Forty Acres Pharmacy.

**Table 1. DPBS formulation**

| Components | Molecular | Concentration (mg/L) | mM |
|---|---|---|---|
| Inorganic Salts | | | |
| Potassium Chloride (KCl) | 75 | 200 | 2.666667 |
| Potassium Phosphate monobasic (KH₂PO₄) | 136 | 200 | 1.470588 |
| Sodium Chloride (NaCl) | 58 | 8,000 | 137.93103 |
| Sodium Phosphate dibasic (Na₂HPO₄-7H₂O) | 268 | 2,160 | 8.059702 |

Two types of nebulizers were utilized. The Aeroneb^{®} Professional Nebulizer System (active vibrating mesh, Aerogen, Galway) is a portable medical device for multiple patient use that is intended to aerosolize physician-prescribed medications for inhalation that are approved for use with a general purpose nebulizer. The EZ Breathe Atomizer (passive vibrating mesh, Nephron Pharmaceuticals Corporation, USA) is a device that is intended to spray liquid medication in aerosol form into the air that a person will breathe. These devices are used by patients with and without mechanical ventilation, or other positive pressure breathing assistance.

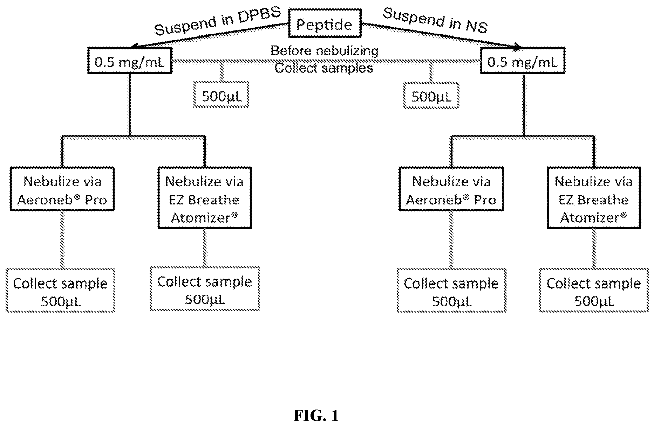

*CSP dispersion preparation* - Test scintillation vials were made according to varying preparations. In a first preparation, 4 mg of scrambled or CSP peptide was weighed and placed in the vial, then 8 mL of PBS or NS was added. In a second preparation, 3 mg of scrambled or CSP-7 peptide was weighed and placed in the vial, then 6 mL of PBS or NS was added. The vials were placed on a magnetic stir and stirred for 30 minutes. The dispersion was then placed in either the Aeroneb Pro or EZ Breathe atomizer. Samples were collected by condensation in chilled vials. A 0.5 mL sample was collected each time and immediately stored at -80°C (see FIG. 1).

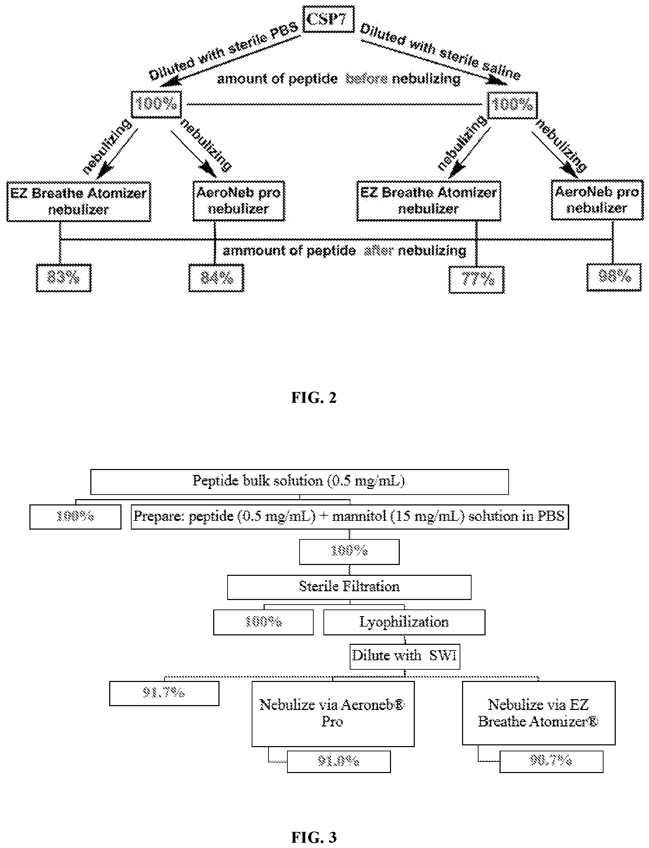

The collected samples were analyzed for peptide content (see FIG. 2). A significant amount of the CSP7 peptide was found to be retained once dispersed in either phosphate buffered saline or normal saline and following nebulization by two vibrating mesh nebulizers. Thus, these studies demonstrate that CSP7 can be formulated for inhalation.

### Example 2 - Peptide Lyophilization and Nebulization

*Formulation preparation* - Lyophilized cake of the CSP 7 mer was prepared using lyophilization. Five hundred µg/mL of the CSP 7 mer solution was prepared as follows: the peptide powder was dissolved in DPBS with minimal volume of ammonium hydroxide (pH 7-8) and 1.5% w/v of mannitol was added in to the solution. The solution was sterile filtered using 0.2 µm SFCA sterile syringe filter (Coming Inc., Corning, NY). Effect of filtration was also tested. One milliliter of the filtrate was filled into a borosilicate glass vial and lyophilized using a VirTis Advantage Lyophilizer (VirTis Company Inc., Gardiner, NY). Lyophilization cycle parameters were set as outlined in Table 2. Primary drying time was varied by number of samples.

**Table 2. Lyophilization cycle parameters**

| Step | Temperature | Hold Time | Pressure | Ramp | |
|---|---|---|---|---|---|
| | °C | min | mTorr | °C/min | min |
| Loading | 5 | 60 | | | |
| Freezing | -55 | 120 | | 0.5 | 120 |
| Annealling | -15 | 120 | | 0.5 | 80 |
| Freezing | -55 | 240 | | 0.5 | 80 |
| Evacuation | -55 | 10 | 100 | | |
| Primary drying | -30 | TBD | 100 | 0.1 | 250 |
| Secondary drying | 30 | 240 | 100 | 0.08 | 720 |

*Nebulization study of the CSP 7 mer peptide* - Lyophilized peptide was reconstituted with sterile water for injection (500 µg/mL). Nebulization was demonstrated using two commercial brands of the vibrating mesh nebulizers to study variation in the efficiency of the nebulizers. The solutions were atomized using Aeroneb^{®} Pro (Aerogen, Mountain View, CA) or EZ Breathe^{®} Atomizer (Model EZ-100, Nephron Pharmaceuticals Corporation, Orlando, FL). Nebulization was stopped after there was no aerosol cloud observed. The condensate of each sample was collected in a polypropylene tube and kept at 5 °C until analyzed. The amount of peptide in each sample was analyzed using high performance liquid chromatography (HPLC) and the degradation products was detected using liquid chromatography mass spectroscopy (LC/MS).

*Chromatographic conditions* - Samples were analyzed using a Dionex 3000 high performance liquid chromatography (HPLC) system (Thermo Fisher Scientific, Fair Lawn, NJ) with a wavelength of 220 nm. The 7 mer peptide was eluted by Phenomenex^{®} Luna 5µ C18(2) 100 Å, 150 mm × 4.6 mm (Phenomenex^{®}, Torrace, CA) column at a flow rate of 1.0 mL/min. Two mobile phases were phase A (0.1% trifluoroacetic acid in water) and mobile phase B (0.09% trifluoroacetic acid in a mixture of 20:80, water and acetonitrile). The HPLC gradient consisted of 25-35% mobile phase B in 20 min was run at ambient temperature. Twenty µL of each sample was injected onto the column and chromatograms were acquired at the detection wavelength of 220 nm. Duplicate determinations were made for each of the samples. The same samples were tested on the LC/MS to determine the degradation products as well.

*Effect of formulation, sterile filtration, lyophilization and nebulization on uPA activity* - Lyophilized peptide was easily dissolved in water. FIG. 3 shows the chemical assay of the peptide. Formulation composition, sterile filtration, or nebulization did not have an influence on the percent recovery of the peptide. Both nebulizers gave similar results.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
U.S. Patent Nos. 5,299,566; 5,889,155; 6,357,671; 6,354,516; 6,241,159; 6,044,841; 6,041,776; 6,016,974; 5,823,179; 5,797,389; 5,660,166; 5,355,872; 5,284,133; 5,277,175; 6,921,020; 6,926,208; 6,968,840; 6,978,941; 7,040,549; 7,083,112; 7,104,463; 8,697,840; 7,332,469 and 7,360,536
U.S. Published Patent Application Nos. 20020020412; 2009/0304666; 20090134235 and 20020020409
International (PCT) Publn. No. WO/2014/145389
Kyte and Doolittle, A simple method for displaying the hydropathic character of a protein, J. Mol. Biol., 157(1):105-32, 1982.
McLean et al. (2000) Anal Chem 72:4796-804, Fults et al. (1991) J Pharm Pharmacol 43:726-8, and Vecellio None et al. (2001) J Aerosol Med 14:107-14.
Shetty S, Gyetko M and Mazar A. Role of urokinase in p53 protein expression: participation in apoptosis and tumorigenesis. J Biol Chem 280(30):28133-41, 2005.
Waldrep and Dhand, Advanced nebulizer designs employing vibrating mesh/aperture plate technologies for aerosol generation, Curr. Drug Deliv., 5(2): 114-9, 2008.
Wawrzynczak and Thorpe, Effect of chemical linkage upon the stability and cytotoxic activity of A chain immunotoxins, Cancer Treat Res., 37:239-51, 1988.

Furthermore, the present invention relates to the following items:
1. A composition comprising a nebulized solution of a biologically active caveolin-1 (Cav-1) scaffolding domain polypeptide.
2. The composition of item 1, wherein the Cav-1 scaffolding domain polypeptide comprises the amino acid sequence of FTTFTVT (SEQ ID NO:1); DGIWKASFTTFTVTKYWFYR (SEQ ID NO:2); or NYHYLESSMTALYTLGH (SEQ ID NO:3).
3. The composition of item 2, wherein the Cav-1 scaffolding domain polypeptide is no more than 20 amino acids in length and comprises the sequence of FTTFTVT (SEQ ID NO:1).
4. The composition of item 3, wherein the Cav-1 scaffolding domain polypeptide is no more than 10 amino acids in length and comprises the sequence of FTTFTVT (SEQ ID NO:1).
5. The composition of item 3, wherein the Cav-1 scaffolding domain polypeptide consists of, or consists essentially of, the sequence of FTTFTVT (SEQ ID NO:1).
6. The composition of item 1, wherein the nebulized solution is produced using a vibrating mesh nebulizer.
7. The composition of item 6, wherein the vibrating mesh nebulizer is an active vibrating mesh nebulizer.
8. The composition of item 6, wherein the vibrating mesh nebulizer is a passive vibrating mesh nebulizer.
9. The composition of item 6, wherein the vibrating mesh nebulizer is an Aeroneb^{®} Professional Nebulizer System or an EZ Breathe Atomizer.
10. The composition of item 1, wherein the nebulized solution is produced using a jet nebulizer or an ultrasonic nebulizer.
11. The composition of item 1, wherein the solution is an aqueous solution.
12. The composition of item 11, wherein the solution comprises a physiologically acceptable salt.
13. The composition of item 11, wherein the solution comprises a pH buffering agent.
14. The composition of item 11, wherein the solution is phosphate buffered saline (PBS).
15. The composition of item 11, wherein the solution is essentially free of a pH buffering agent.
16. The composition of item 11, wherein the solution is a sterile saline solution.
17. The composition of item 11, wherein the composition consists essentially of a sterile saline solution and the biologically active Cav-1 scaffolding domain polypeptide.
18. The composition of item 1, wherein the nebulized solution further comprises a biologically active enzyme.
19. The composition of item 18, wherein the enzyme is a plasminogen activator enzyme.
20. The composition of item 19, wherein the plasminogen activator enzyme is tissue plasminogen activator (tPA) or urokinase plasminogen activator (uPA).
21. The composition of item 19, wherein the uPA is a single chain uPA (scuPA).
22. The composition of item 1, wherein the nebulized solution has a median particle size of between about 2.5 µm and 20 µm.
23. The composition of item 22, wherein the nebulized solution has a median particle size of between about 2.5 µm and 8 µm.
24. The composition of item 23, wherein the nebulized solution has a median particle size of between about 3.0 µm and 6 µm.
25. A composition for use in the treatment of treating or preventing a fibrotic condition of the lungs, said composition comprising a composition in accordance with any one of items 1-24.
26. A method of treating or preventing acute lung injury, lung infection or lung disease in a subject comprising administering to the subject an effective amount of a biologically active Cav-1 scaffolding domain polypeptide, said polypeptide being administered in a nebulized solution to the airway of the subject.
27. The method of item 26, wherein the subject has an acute lung injury or infection.
28. The method of item 26, wherein the subject has a chemical-induced lung injury.
29. The method of item 26, wherein the subject has plastic bronchitis, asthma, acute respiratory distress syndrome (ARDS) or inhalational smoke induced acute lung injury (ISALI).
30. The method of item 26, wherein the lung disease is a fibrotic condition of the lungs.
31. The method of item 26, wherein the lung disease is interstitial lung disease.
32. The method of item 26, wherein the lung disease is Idiopathic Pulmonary Fibrosis (IPF) or lung scarring.
33. The method of item 26, wherein the administering comprises nebulizing a solution comprising the Cav-1 scaffolding domain polypeptide.
34. The method of item 33, wherein the nebulizing comprises using an ultrasonic or jet nebulizer to nebulize the solution comprising the Cav-1 scaffolding domain polypeptide.
35. The method of item 33, wherein the nebulizing does not comprise using an ultrasonic or jet nebulizer to nebulize the solution comprising the Cav-1 scaffolding domain polypeptide.
36. The method of item 33, wherein the nebulizing comprises using a vibrating mesh nebulizer to nebulize the solution comprising the Cav-1 scaffolding domain polypeptide.
37. The method of item 36, wherein the vibrating mesh nebulizer is an active vibrating mesh nebulizer.
38. The method of item 36, wherein the vibrating mesh nebulizer is a passive vibrating mesh nebulizer.
39. The method of item 36, wherein the vibrating mesh nebulizer is an Aeroneb^{®} Professional Nebulizer System or an EZ Breathe Atomizer.
40. The method of item 36, wherein the nebulizing comprises:
   (i) obtaining a lyophilized polypeptide composition, comprising a Cav-1 scaffolding domain polypeptide;
   (ii) reconstituting the lyophilized enzyme composition in an aqueous solution to provide polypeptide solution; and
   (iii) nebulizing the polypeptide solution.
41. The method of item 26, comprising administering a nebulized solution in accordance with any one items 1-25.
42. The method of item 26, further comprising administering a biologically active plasminogen activator enzyme to the subject.
43. The method of item 42, wherein the plasminogen activator enzyme is administered in a nebulized solution to the airway of the subject.
44. The method of item 2, wherein the plasminogen activator enzyme is tPA or uPA.
45. The method of item 44, wherein the plasminogen activator is scuPA.
46. A method of preparing a biologically active Cav-1 scaffolding domain polypeptide for administration to a subject's airway comprising nebulizing a solution comprising the Cav-1 scaffolding domain polypeptide to provide a nebulized solution.
47. The method of item 46, wherein the Cav-1 scaffolding domain polypeptide comprises the amino acid sequence of FTTFTVT (SEQ ID NO:1); DGIWKASFTTFTVTKYWFYR (SEQ ID NO:2); or NYHYLESSMTALYTLGH (SEQ ID NO:3).
48. The method of item 47, wherein the Cav-1 scaffolding domain polypeptide is no more that 20 amino acids in length and comprises the sequence of FTTFTVT (SEQ ID NO:1).
49. The method of item 48, wherein the Cav-1 scaffolding domain polypeptide is no more that 10 amino acids in length and comprises the sequence of FTTFTVT (SEQ ID NO:1).
50. The method of item 47, wherein the Cav-1 scaffolding domain polypeptide consists of, or consists essentially of, the sequence of FTTFTVT (SEQ ID NO:1).
51. The method of item 46, wherein nebulizing the solution is by using a vibrating mesh nebulizer.
52. The method of item 51, wherein the vibrating mesh nebulizer is an active vibrating mesh nebulizer.
53. The method of item 51, wherein the vibrating mesh nebulizer is a passive vibrating mesh nebulizer.
54. The method of item 51, wherein the vibrating mesh nebulizer is an Aeroneb^{®} Professional Nebulizer System or an EZ Breathe Atomizer.
55. The method of item 46, wherein the nebulizing the solution comprises using an ultrasonic or jet nebulizer.
56. The method of item 46, wherein the nebulizing the solution does not comprise using an ultrasonic or jet nebulizer.
57. The method of item 46, wherein the solution is an aqueous solution.
58. The method of item 46, further defined as a method of producing a a composition in accordance with any one of items 1-25.
59. The method of item 46, wherein nebulizing comprises:
   (i) obtaining a lyophilized polypeptide composition, comprising a Cav-1 scaffolding domain polypeptide;
   (ii) reconstituting the lyophilized enzyme composition in an aqueous solution to provide polypeptide solution; and
   (iii) nebulizing the polypeptide solution.
60. A nebulized polypeptide solution produced by a method according to any one of items 46-59.
61. The method of any one of items 46-59, further comprising administering the nebulized solution to the airway of a subject in need thereof.
62. The composition of item 1, further comprising a lyoprotectant.
63. The composition of item 62, wherein the lyoprotectant is mannitol.
64. The composition of item 62, wherein the mannitol is present in a ratio of 100:1 to 5:1 w/w relative to the Cav-1 scaffolding domain polypeptide.
65. A lyophilized composition comprising a biologically active caveolin-1 (Cav-1) scaffolding domain polypeptide and at least a first lyoprotectant agent, wherein the Cav-1 scaffolding domain polypeptide remains soluble when exposed to an aqueous solution.

## Claims

1. A composition comprising a caveolin-1 (Cav-1) scaffolding domain polypeptide comprising of the sequence FTTFTVT (SEQ ID NO: 1) and a pharmaceutically acceptable carrier for use in the treatment or prevention of injury, infection, or disease of the lungs in a subject, wherein the composition is administered to the airway of the subject as a nebulized solution.

2. The composition for use according to claim 1, wherein the nebulized solution has a median particle size of between 2.5 µm and 20 µm.

3. The composition for use according to claims 1 or 2, wherein the nebulized solution is produced using a vibrating mesh nebulizer.

4. The composition for use according to claims 1 or 2, wherein the nebulized solution is produced using a jet nebulizer or an ultrasonic nebulizer.

5. The composition for use according to any one of claims 1 to 4, wherein the composition and/or nebulized solution comprises a lyoprotectant.

6. The composition for use according to claim 5, wherein the lyoprotectant comprises dextrose, mannose, galactose, fructose, lactose, sucrose, maltose, sorbitol, or mannitol, preferably wherein the lyoprotectant is mannitol.

7. The composition for use according to any one of claims 1 to 6, wherein the composition and/or nebulized solution comprises a pH buffering agent.

8. The composition for use according to any one of claims 1 to 7, wherein the composition and/or nebulized solution is a sterile and/or normal saline solution or a phosphate buffered saline solution.

9. The composition for use according to claims 1 or 2, wherein the composition consists essentially of a sterile and/or normal saline solution and the Cav-1 scaffolding domain polypeptide.

10. The composition for use according to any one of claims 1 to 9, wherein the subject has an acute lung injury, chemical-induced lung injury, infection of the lungs, or infectioninduced lung injury.

11. The composition for use according to any one of claims 1 to 10, wherein the subject has plastic bronchitis, asthma, chronic obstructive airway/pulmonary disease (COPD), acute respiratory distress syndrome, inhalational smoke induced acute lung injury, bronchiectasis, inhalational toxin-induced airway disease, exposure to mustard gas, exposure to particulate matter, bronchiolitis obliterans, bronchiolitis obliterans organizing pneumonia, collagen vascular lung disease, interstitial lung disease, or drug induced lung disease and accelerated pulmonary fibrosis.

12. The composition for use according to claim 11, wherein the interstitial lung disease is idiopathic pulmonary fibrosis or sarcoidosis.

13. The composition for use according to any one of claims 1 to 12, wherein the subject has a fibrotic condition of the lungs.

14. The composition for use according to any one of claims 1 to 13, wherein the caveolin-1 polypeptide is administered at a dose of about 5 µg/kg of body weight to about 500 mg/kg of body weight.

15. The composition for use according to any one of claims 1 to 14, wherein the caveolin-1 polypeptide consists of the sequence FTTFTVT (SEQ ID NO: 1).


SEQUENCE LISTING

<110>  BOARD OF REGENTS, THE UNIVERSITY OF TEXAS SYSTEM

<120>  POLYPEPTIDE THERAPEUTICS AND USES THEREOF

<130>  AA2370 EP/1 S3

<150>  US 62/126,039
<151>  2015-02-27

<160>  3

<170>  PatentIn version 3.5

<210>  1
<211>  7
<212>  PRT
<213>  Homo sapiens

<400>  1

Phe Thr Thr Phe Thr Val Thr
1               5

<210>  2
<211>  20
<212>  PRT
<213>  Homo sapiens

<400>  2

Asp Gly Ile Trp Lys Ala Ser Phe Thr Thr Phe Thr Val Thr Lys Tyr
1               5                   10                  15

Trp Phe Tyr Arg
            20

<210>  3
<211>  17
<212>  PRT
<213>  Artificial sequence

<220>
<223>  Synthetic polypeptide

<400>  3

Asn Tyr His Tyr Leu Glu Ser Ser Met Thr Ala Leu Tyr Thr Leu Gly
1               5                   10                  15

His